Europäisches Patentamt

European Patent Office

Office européen des brevets

(11) Veröffentlichungsnummer: **0 185 335 B1**

# EUROPÄISCHE PATENTSCHRIFT

(45) Veröffentlichungstag der Patentschrift: **24.04.91**

(51) Int. Cl.⁵: **C12Q 1/37**, G01N 33/564

(21) Anmeldenummer: **85115979.8**

(22) Anmeldetag: **14.12.85**

(54) **Verfahren zum Nachweis des Vorliegens einer Allergie und zum spezifischen Nachweis des für die Allergie verantwortlichen Allergens.**

(30) Priorität: **21.12.84 DE 3446714**

(43) Veröffentlichungstag der Anmeldung:
**25.06.86 Patentblatt 86/26**

(45) Bekanntmachung des Hinweises auf die
Patenterteilung:
**24.04.91 Patentblatt 91/17**

(84) Benannte Vertragsstaaten:
**AT BE CH DE FR GB IT LI LU NL SE**

(56) Entgegenhaltungen:
**EP-A- 0 090 359**
**DE-A- 3 147 763**

**CHEMICAL ABSTRACTS, Band 60, Nr. 2, 20. Januar 1964, Seiten 52-59, Zusammenfassung 1987a, Spalte 1987, Columbus, Ohio, US; L.A. MOUNTER et al.: "Proteases of human leukocytes"**

(73) Patentinhaber: **BOEHRINGER MANNHEIM GMBH**
**Sandhofer Strasse 116**
**W-6800 Mannheim 31(DE)**

(72) Erfinder: **Wilhelms, Otto-Henning, Dr. rer. nat.**
**Odenwaldstrasse 52/2**
**W-6941 Weinheim-Rittenweier(DE)**

## Beschreibung

Die Erfindung betrifft ein verbessertes Verfahren zum Nachweis des Vorliegens einer Allergie und zum spezifischen Nachweis des für die Allergie verantwortlichen Allergens. Ebenso eignet sich dieses Verfahren zur Diagnostik anderer Erkrankungen, an deren Pathomechanismus eine Stimulus-induzierte Freisetzung von Mediatoren aus Mastzellen und Basophilen beteiligt ist. Ferner kann das Verfahren angewendet werden für ein automatisierbares Screeningverfahren von Pharmaka, welche die Stimulus-induzierte Mediatorfreisetzung aus Basophilen, Mastzellen und Phagozyten unterbinden und somit für eine Therapie allergischer und entzündlicher Erkrankungen geeignet sind.

Bei Allergikern finden sich im Blut erhöhte IgE-Spiegel. IgE findet sich außerdem gebunden an Mastzellen und Basophilen. Die allergische Reaktion wird eingeleitet durch Bindung des spezifischen Allergens an dafür spezifische Zell-gebundene IgE-Moleküle.

Vielfach wird angenommen, daß diese Bindung des stimulierenden Allergens an IgE über die sogenannte Verbrückung benachbarter IgE in komplexer Reaktion die Degranulation von basophilen Leukozyten bewirkt. Die Degranulation setzt bekanntermaßen Histamin, Proteasen, Metabolite der Arachidonsäure, Phosphatasen und andere Enzyme frei.

In der DE-A 31 47 763 wird deshalb für die Diagnostik allergischer Erkrankungen vorgeschlagen, das Blut von möglicherweise allergischen Personen in eine die weißen Blutkörperchen enthaltende Plasmaschicht und eine Schicht mit den roten Blutkörperchen zu trennen, die Plasmaschicht zu gewinnen, mit einer Indikatorlösung zu versetzen und teilweise als Kontrolle zurückzubehalten und teilweise mit dem bzw. den zu prüfenden Allergenen zu versetzen. Nach einer mehr oder weniger langen Inkubationszeit wird die Indikatorreaktion entweder direkt oder nach Zusatz weiterer Reagenzien photometrisch bestimmt und durch Vergleich mit den Ausgangswerten bzw. dem Wert der Kontrollösung die Enzymaktivität bestimmt. Als mögliche zu messende Enzyme werden Proteasen und alkalische Phosphatase genannt, welche durch die allergische Reaktion aktiviert werden sollen. Schwierigkeiten bereitet bei diesem Verfahren, daß Allergene, die selbst eine Protease oder Phosphataseaktivität aufweisen, mit diesem Test nicht nachgewiesen werden können und andererseits das Serum auch gesunder Patienten eine gewisse Protease- und Phosphataseaktivität aufweist und dadurch auch die Kontrollösung eine Reaktion zeigt, die von der der Meßlösung abgezogen werden muß, um auf den spezifisch durch die Allergie hervorgerufenen Wert zu kommen, was bekanntlich zu einer erheblichen Fehlerbreite führt.

In der DE-A 32 11 254 wird deshalb vorgeschlagen, in Analogie zu dem bekannten fluorimetrischen Histaminfreisetzungstest die Leukozyten von dem Serum zu trennen, anhaftende Serumreste durch mehrfaches Waschen mit einem geeigneten Puffergemisch zu entfernen und die Zellen in einem solchen Puffer wieder zu resuspendieren. Die so erhaltene Suspension wird danach mit einem Allergen oder mit einer Lösung von anti-IgE Antikörpern versetzt und in einem weiteren Schritt durch Zugabe einer Calciumchloridlösung die Freisetzung von Histamin bzw. Protease stimuliert. Nach einer definierten Inkubationszeit wird die Reaktion durch Zugabe von EDTA-Lösung gestoppt, die Zellen sedimentiert und in dem Überstand, anstelle der vorbekannten, aufwendigen fluorimetrischen Messung von Histamin, durch Zugabe einer Indikatorlösung die durch das Allergen freigesetzte Proteaseaktivität gemessen. Das Verfahren kommt somit ohne Geräte bzw. mit einem einfachen Photometer aus. Obwohl dieses Verfahren wesentlich weniger störanfällig ist als die fluorimetrische Histaminmessung, erweist es sich durch die notwendige Anzahl von Verfahrensschritten für den Routinetest als umständlich.

Aufgabe der vorliegenden Erfindung war es daher, ein Verfahren für die Diagnostik allergischer Erkrankungen zu schaffen, mittels dem die vorgenannte zelluläre Reaktion auf Allergene einfacher und schneller durchgeführt werden kann und möglichst die Empfindlichkeit der Nachweisreaktion noch gesteigert wird. Dasselbe Verfahrensprinzip sollte sich auch für die Diagnose anderer Erkrankungen eignen, wie z.B. der rheumatischen Arthritis, der Arzneimittelüberempfindlichkeit (z.B. Penicillin-Unverträglichkeit) oder der Unverträglichkeit bei Dialyse-pflichtigen Patienten, an deren Pathomechanismus eine Stimulus-induzierte Mediatorfreisetzung aus Basophilen und Mastzellen beteiligt ist.

Diese Aufgabe wird erfindungsgemäß gelöst durch ein Verfahren zum Nachweis des Vorliegens einer Allergie und zum spezifischen Nachweis des für die Allergie verantwortlichen Allergens, wobei Leukozyten der zu untersuchenden Probe mit einem natürlichen oder synthetischen Allergen oder mit einem anderen Stimulationsfaktor wie anti-IgE Antikörper, RNS, DNS oder Immunkomplexen in wässrigem Medium inkubiert und die freigesetzte Protease mit einem geeigneten Reagenz nachgewiesen wird, dadurch gekennzeichnet, daß ein Allergen bzw. ein anderer Stimulationsfaktor, ein Puffer, ein geeigneter Indikator und ein Calciumsalz miteinander vermischt und eine Suspension der zu untersuchenden Leukozyten zugefügt und die aus den Zellen freigesetzte Proteaseaktivität nach einer Inkubationszeit kinetisch an der Zunahme der Chromophor-Konzentration gemessen wird. Analog dient zum Nachweis einer rheumatischen Arthritis die Freisetzung von Protease nach Zusatz von DNS, RNS oder Immunkomplexen zur Leukozytensuspension.

Gegenstand der Erfindung ist ferner ein für das vorstehende Verfahren geeignetes Reagenz, welches aus dem oder den Stimulationsfaktor(en) wie Allergenen, anti-IgE Antikörpern, DNS, RNS oder Immunkomplexen, einem Puffer, einem Chromogen und einem Calziumsalz besteht.

Da für die Reaktion nur geringe Reagenz- und Testsubstanzmengen (0,2-1 ml pro Probe) benötigt werden, läßt sich die Reaktion vorzugsweise auf einer Mikrotiterplatte durchführen, wobei die Reagenzien vorzugsweise in fester, insbesondere lyophilisierter Form und definierter Menge in den Behältern der Mikrotiterplatte vorliegen und die Reaktionslösung durch Zugabe der Leukozytensuspension rekonstituiert wird. Ebenso können die Reagentien auf einen saugfähigen Träger, beispielsweise ein Papier-, Kunststoff- oder Glasfaservlies, einen quellfähigen Kunststoffilm oder ein Gel imprägniert sein und in dieser Form in das Reaktionsgefäß gegeben werden. Der imprägnierte Träger kann jedoch auch direkt mit der Leukozytensuspension befeuchtet und zur Reaktion gebracht werden, wie es für andere trockenchemische Reaktionen üblich ist. Die chromogene Reaktion kann entweder visuell oder bei höheren Anforderungen an die Genauigkeit mit einem Photometer vermessen werden. Für den Durchsatz größerer Probenmengen läßt sich dieses Verfahren selbstverständlich in an sich bekannter Weise automatisieren. Deshalb ist das Verfahren auch besonders geeignet für ein Pharmakascreening von Antiallergika, welche die Stimulus-induzierte Degranulation von Mastzellen und Basophilen hemmen. Dasselbe Testprinzip ist auch geeignet zum Nachweis der Enzymfreisetzung aus phagozytierenden Zellen nach Zusatz von z.B. opsonisiertem Zymosan und somit zur Entdeckung von entzündungshemmenden Substanzen.

Es erscheint überraschend, daß es möglich ist, sämtliche für die Freisetzung und den Nachweis der Protease notwendigen Reagenzien in einer einheitlichen Mischung vorzulegen und in einem einfachen Pipettierschritt lediglich die Leukozytensuspension zuzufügen, da einerseits angenommen wurde, daß die Indikatoren in der bisher üblichen Konzentration von $10^{-4}$ - $10^{-3}$ Mol/l eine spontane Freisetzung von Protease aus Leukozyten bewirken, wenn diese Reaktion nicht vorher durch Zugabe von komplexbildenden Mitteln wie EDTA inhibiert wird und andererseits bekannt war, daß es nicht möglich ist, die zur Freisetzung der Protease aus den basophilen Leukozyten notwendigen Calzium- oder Magnesiumionen vor der Zugabe der stimulierenden Allergene der Leukozytenlösung zuzufügen, da die Calziumionen in diesem Fall eine Veränderung der Leukozytenmembran bewirken, die einen Angriff der Allergene inhibiert.

Wie nun gefunden wurde, bewirkt der Zusatz der Chromogene in einer Menge von $10^{-4}$ bis $10^{-3}$ Mol/l keine unspezifische, spontane Freisetzung von Protease bzw. eine so geringe, daß sie den Test nicht stört. Andererseits wird durch diesen Zusatz überraschenderweise die durch das Allergen freigesetzte Protease in noch unbekannter Weise stabilisiert, so daß bei Zusatz der gleichen Allergenmenge gegenüber den bisher bekannten Umsetzungen eine deutlich höhere Proteaseaktivität gemessen wird. Es wird angenommen, daß bei gleichzeitiger Zugabe von Allergen und Calziumionen die Allergene so rasch an die aktiven Stellen der Zellmembran binden, daß eine Verminderung der Reaktivität durch die Calziumionen nicht eintritt und diese lediglich den gewünschten Proteasetransport durch die Zellmembran katalysieren.

Die nachfolgenden Angaben zur Durchführung des Verfahrens gelten allgemein für alle dessen Varianten. Sie werden dargestellt am Beispiel der Anwendung des Verfahrens für die Allergie-Diagnose. Für das erfindungsgemäße Verfahren werden je nach Art des Allergens und der Stärke der allergischen Reaktion, Allergene in einer Menge von 0,01 bis 100 mg/l, insbesondere 1 - 10 mg/l Reaktionslösung eingesetzt.

Calziumionen sowie gegebenenfalls Magnesiumionen werden vorteilhaft in einer Konzentration von 0,5 - 5 x $10^{-3}$ Mol/l, insbesondere 3 - 4 x $10^{-3}$ Mol/l eingesetzt. Diese Ionen werden üblicherweise in Form wasserlöslicher Salze, insbesondere als Chloride, Sulfate, Acetate oder Citrate eingesetzt.

Die Reaktion wird in einer auf ein pH von 6,3 - 9, insbesondere 7,0 - 8 und vorzugsweise 7,5 gepufferten Lösung durchgeführt. Als Puffer wird vorzugsweise der gleiche Puffer verwendet, der auch zum Waschen und Suspendieren der Leukozyten verwendet wird, d.h. ein physiologischer Puffer, der durch Zusätze von Natriumchlorid, Kaliumchlorid, Glucose oder anderen physiologisch verträglichen Substanzen isotonisch gestellt wird und eine Konzentration von etwa 5 x $10^{-3}$ bis $10^{-1}$ Mol/l Puffersubstanz besitzt. Als Puffersubstanz werden Trispuffer, Phosphatpuffer, Glycinpuffer oder andere physiologisch verträgliche Puffer, ggf. auch als Mischung, verwendet, deren Pufferkapazität auf den vorgenannten pH-Bereich eingestellt werden kann und die in den oben angegebenen Konzentrationen mit den Calzium- bzw. Magnesiumionen keine unlöslichen Salze bilden. Im Rahmen der Anmeldung werden diese Puffersysteme ebenfalls als "Puffer" bezeichnet.

Zur Stabilisierung der Leukozyten kann diese Lösung zusätzlich noch Gelatine oder andere makromolekulare Stoffe mit zellstabilisierenden Eigenschaften, wie sie jedem Fachmann gut bekannt sind, enthalten.

Die Reaktion wird üblicherweise zwischen Raumtemperatur und 37° C durchgeführt, wobei den physiologischen Bedingungen entsprechend eine Temperatur von 35 - 37° C bevorzugt ist. Bei dieser Temperatur

3

ist normalerweise nach 15 - 60 Minuten Inkubation so viel Proteinase freigesetzt, daß die Messung erfolgen kann. Bei geringer Allergenkonzentration und schwacher Freisetzungsreaktion kann die Inkubationszeit jedoch erhöht werden. In diesem Fall wird die Meßreaktion bevorzugt bei Raumtemperatur durchgeführt, da die Enzyme, wie wir festgestellt haben, bei dieser Temperatur auch über längere Zeiträume (über 100 h!) stabil sind und eine lineare Reaktions-Kinetik ergeben. Bei starker Freisetzungsreaktion muß darauf geachtet werden, daß keine Substanzverarmung eintritt, weil sonst die Farbreaktion nicht mehr linear verläuft. Ein Messen bei 37° C beschleunigt in diesen Fällen die Enzymreaktion. Messungen bei Raumtemperatur lassen sich jedoch auch vorteilhaft durchführen.

Aufgrund der einfachen Versuchsdurchführung, die nur noch einen Pipettierschritt für die Zugabe der Leukozyten zu den fertigen Reagenzmischungen erfordert, ist das erfindungsgemäße Verfahren insbesondere geeignet für größere Reihenversuche, bei denen die Empfindlichkeit einer vorgegebenen Leukozytensuspension gegen verschiedene Allergene oder andere Stimuli geprüft werden soll und andererseits geeignet, um unbekannte Substanzen auf ihre antiallergene Wirkung zu prüfen. Dabei werden diese Substanzen entweder der Reagenz- oder der Leukozytensuspension in geeigneter Konzentration zugemischt und die Verringerung der Proteasefreisetzung gegenüber einer nicht mit der fraglichen Substanz versetzten Kontrollösung bestimmt wird.

Als chromogene Substanzen im Sinne der vorstehenden Erfindung können alle diejenigen verwendet werden, die im erfindungsgemäßen pH-Bereich mit Protease reagieren und ein visuell, photometrisch oder auch fluorimetrisch detektierbares Produkt erzeugen. Gebräuchliche Substrate sind beispielsweise N-Benzoylarginin-p-nitranilid, N-(3-Carboxypropionyl)-phenylalanin-p-nitranilid oder durch Peptide substituierte p-Nitroanilide, wie sie beispielsweise in den DE-A 25 27 932, 25 52 570, 26 29 067 und 32 11 254 beschrieben sind. Andere Reagenzien mit chromophoren bzw. fluoreszierenden Resten, die durch abspaltbare Peptide substituiert sind, sind in der DE-A 29 36 543, DE-A 30 17 721, DE-A 28 54 987 und EP-A 78 703 beschrieben. Unter Chromogen im Sinne dieser Anmeldung wird dabei nicht nur das Substrat selbst, sondern auch die sonstigen zur Ausbildung oder Farbbildung ggf. noch notwendigen Reagenzien verstanden, wie sie beispielsweise in den vorstehenden Patentschriften beschrieben sind. Besonders genannt seien Reaktionsbeschleuniger, Stabilisatoren, Oxidationshilfsmittel, Farbkuppler, Kontrastfärbemittel, viskositätsregulierende Stoffe und Netzmittel.

In den folgenden Beispielen ist die Erfindung näher ausgeführt, ohne daß dadurch der Umfang eingeschränkt werden soll.

**Beispiel 1**

1.1 Leukozyten Gewinnung

60 ml venöses Blut eines Spenders wurde mit 30 ml Dextran-Gemisch (Dextran mit mittlerem MG 75000 2 %; d-Glucose 2 %; EDTA 20 mmol/l; Gelatine 0,07 %) vorsichtig in einem Polyäthylen-Becherglas gemischt und in einen silikonisierten Scheidetrichter überführt. Nach 60 bis 90 Minuten Stehenlassen bei Raumtemperatur wurde der die Leukozyten enthaltende Überstand (ca. 50 ml) abgetrennt und mit 50 ml Tris-Puffer-Gemisch, pH 7,6 (Tris 22 mmol/l; NaCl 0,12 mol/l; KCl 5 mmol/l; Gelatine 0,05 %; EDTA 1 mmol/l; D-Glucose 0,1 %) versetzt und 15 Minuten bei 800 g zentrifugiert. Nach Dekantieren wurden die Zellen 2mal gewaschen, indem sie jeweils mit nochmals 50 ml des gleichen Puffergemisches, pH 7,6, suspendiert und jeweils 15 Minuten bei 800 g zentrifugiert wurden. Die Zellen wurden in 10 ml des gleichen Puffergemisches suspendiert und durch Verdünnen mit weiterem Puffer auf $10^7$ Zellen/ml eingestellt.

1.2 Reagenz-Mischung

| 10 ml | Anti-IgE-Lösung (Behring Werke Nr. OTNP 04/05 1:500 mit Tris-Puffer pH 7,6 wie in 1.1 verdünnt. |
|---|---|
| 10 ml | Ca Cl₂-Lösung (4 mmol/l in isotonischer NaCl-Lösung, pH 7,6) |
| 20 ml | Chromozym TH-Lösung 0,75 mmol/l (1 Flasche Boehringer Mannheim, Best.-Nr.199664 in 10 ml H₂O lösen) werden gründlich gemischt und bis zur Verwendung gelagert. |

1.3 Proteinase-Stimmulierung mit anti-IgE-Antikörpern

Je 0,5 ml der Leukozytensuspension werden zu 0,5 ml der Reagenzmischung gegeben, bei 37°C inkubiert und die Extinktion bei 405 nm nach 45, 90 und 180 min. gemessen.

Um die unspezifische Zersetzung zu ermitteln, wird eine entsprechende Kontrolle ohne den anti-IgE-Zusatz, aber allen übrigen Bestandteilen der Reagenzlösung, mitgeführt. Aus den Extinktionsdifferenzen zwischen Meß- und Kontrolllösung läßt sich die Proteinasefreisetzung aus den Leukozyten durch das Stimmulans bestimmen.

### a) Blut von "Nicht-Allergikern"

| Mittelwerte aus 3 Messungen | 45 min | 90 min | 180 min |
|---|---|---|---|
| Kontrolle | 0,015 | 0,042 | 0,097 |
| anti-IgE | 0,062 | 0,170 | 0,382 |
| $\triangle E_{405}$ | 0,047 | 0,128 | 0,285 |

### b) Blut von Allergikern (Heuschnupfen)

| | 45 min | 90 min | 180 min |
|---|---|---|---|
| Kontrolle | 0,025 | 0,058 | 0,122 |
| Anti-IgE | 0,305 | 0,857 | 1,743 |
| $\triangle E_{405}$ | 0,280 | 0,799 | 1,621 |

### 1.4 Protease-Stimmulierung mit Gras-Allergen

Unter den gleichen Bedingungen wie bei 1.3, jedoch mit einem Reagenz, das 10 ml Allergenextrakt aus Gräserpollen ($10^{-5}$ g/ml Allergen in Tris-Puffer pH 7,6 gemäß 1.1) enthält, werden Blutproben von gesunden und allergischen Probanden verglichen. a) Blut von Nicht-Allergikern

| Mittelwerte aus 3 Messungen | 45 min | 90 min | 180 min |
|---|---|---|---|
| Kontrolle | 0,018 | 0,037 | 0,092 |
| Allergen | 0,037 | 0,045 | 0,109 |
| △ E405 | 0,019 | 0,008 | 0,017 |

b) <u>Blut von Allergikern</u> (Heuschnupfen)

| Mittelwerte aus 3 Messungen | 45 min | 90 min | 180 min |
|---|---|---|---|
| Kontrolle | 0,020 | 0,048 | 0,082 |
| Allergen | 0,180 | 0,424 | 0,864 |
| △ E405 | 0,160 | 0,376 | 0,782 |

## 1.5 Interpretation der Ergebnisse

Es zeigt sich aus dem Vergleich der Reaktionen, daß die durch den Puffer bewirkte unspezifische Farbstoffbildung deutlich geringer ist, als die durch freigesetzte Protease bewirkte.

Anti-IgE-Antikörper bewirken auch mit den Leukozyten gesunder Probanden eine gewisse Proteasefreisetzung, die jedoch schwächer ist als bei Allergikern, so daß dieser Test als unspezifischer Test auf allergische Reaktionen verwendet werden kann.

Die schwache Reaktion normaler Leukozyten gegenüber Allergenen unterscheidet sich wiederum signifikant von der starken Reaktion von allergisierten Leukozyten, die eine einwandfreie Bestimmung der Allergene ermöglicht.

## Beispiel 2

Einfluß der Calziumionenzugabe auf die Histamin- und Proteinase-Freisetzung

Zum Nachweis des Einflußes der Reihenfolge der Zufügung der Reagentien wurde der in 1.3 a) aufgeführte Test wiederholt, die Reagenzbestandteile gemäß 1.2 jedoch in der folgenden Reihenfolge zugefügt.

a) 0,5 ml Zellsuspension gemäß 1.1 wird mit 0,125 ml CaCl$_2$-Lösung versetzt und nach 10 min 0,125 ml anti-IgE-Lösung zugefügt.

b) 0,5 ml Zellsuspension werden mit einem Gemisch aus 0,125 ml CaCl$_2$-Lösung und 0,125 ml anti-IgE Lösung versetzt.

c) 0,5 ml Zellsuspension werden mit 0,125 ml anti-IgE-Reagenzlösung versetzt und nach 1 min 0,125 ml CaCl$_2$-Lösung zugefügt.

Als Kontrolle wird jeweils 0,5 ml Tris-Puffer in gleicher Weise mit den Reagenzlösungen gemischt.

Alle Ansätze werden 60 min bei 37°C inkubiert, die Proteinasefreisetzung durch Zufügen von 0,5 ml EDTA-Lösung (0,1 mol/l in 0,15 M Tris-Puffer pH 7,6) gestoppt und die Zellen abzentrifugiert. Im zellfreien Überstand wird mit automatisierter Fluorimetrie das freigesetzte Histamin und in einem abgetrennten Teil durch Zugabe von Chromozym TH (5 x 10$^{-4}$ M) photometrisch die nach weiteren 60 min bei 37°C freigesetzte Proteaseaktivität gemessen. Die in der folgenden Tabelle aufgeführten Werte sind aus 3-6 Messungen gemittelt.

| | Histamin % von Gesamtgehalt | | | Proteinase ($\Delta E_{405}$) | | |
|---|---|---|---|---|---|---|
| | a | b | c | a | b | c |
| Kontrolle | 7 | 4 | 6 | 0,040 | 0,016 | 0,017 |
| Probe | 14 | 40 | 35 | 0,084 | 0,271 | 0,227 |
| Differenz | 7 | 36 | 29 | 0,044 | 0,255 | 0,210 |

Der Versuch zeigt deutlich, daß ein vorzeitiges Zufügen von CaCl$_2$ die Freisetzung hemmt.

## Beispiel 3

Einfluß der Chromogenzugabe auf die Histamin- und Proteinase-Freisetzung

Um den Einfluß der Chromogenzugabe zu demonstrieren, wurden folgende Versuche durchgeführt:

a) 0,5 ml Zellsuspension werden mit einem Gemisch aus 0,125 ml CaCl$_2$-Lösung und 0,125 ml anti-IgE-Lösung versetzt. Nach 60 min Inkubation bei 37°C wird die Reaktion mit 0,125 ml EDTA-Lösung gestoppt. Im Überstand wird nach Zentrifugation der Histamin- und Proteinasegehalt bestimmt. Die Proteinase wird durch Zugabe von 0,125 ml Chromozym TH-Lösung und weiteres Inkubieren (60 min / 37°C) und Messen der Extinktionsdifferenz bei 405 nm gemessen.

b) 0,5 ml Zellsuspension werden mit 0,5 ml Reagenz gemäß 1.2 vermischt, 60 min bei 37°C inkubiert, der Histamingehalt (in einem zellfreien Teil) bestimmt und ein erster Extinktionswert bei 405 nm gemessen. Nach weiterem Inkubieren bei 37°C/60 min wird die zweite Extinktion gemessen und aus der Extionktionszunahme der Proteinasegehalt bestimmt.

c) In einem weiteren Ansatz wird wie unter a) gearbeitet jedoch die Zellen aus der zur Proteinasebestimmung dienenden Probe nicht abgetrennt.

| | Histamin %  | | | Proteinase ($\Delta$ E405) | | |
|---|---|---|---|---|---|---|
| | a | b | c | a | b | c |
| Kontrolle | 4 | 11 | 6 | 0,02 | 0,07 | 0,03 |
| Probe | 24 | 40 | 35 | 0,18 | 0,51 | 0,20 |
| Differenz | 20 | 29 | 29 | 0,16 | 0,44 | 0,17 |

Es zeigt sich aus dem Vergleich der vorstehenden Meßwerte, daß die Testvariante b) ein deutlich stärkeres Proteinasemeßsignal ergibt als bei c), während die Histaminfreisetzung etwa gleich hoch ist. Es wird daraus geschlossen, daß in Gegenwart des chromogenen Substrats die freigesetzte Proteinase stabilisiert bzw. vor einer Inaktivierung durch andere Testbestandteile geschützt wird.

**Beispiel 4**

Vergleich der Allergen- und anti-IgE-induzierten Proteinasefreisetzung aus Leukozytensuspension verschiedener Probanden mit Allergieverdacht

Meßbeispiel in Mikrotiterplatte; 0.3 ml-Ansätze ~ 1.4 x 10$^7$ Zellen/ml (Microtiterplatten Nunclon 96U)
Konzentrationen: verschiedene Allergene 10 µg/ml; anti-IgE-Antikörperlösung: 1:8000-Verdünnung, sonstige Reagenzien wie in Beispiel 3.
Proteinaseaktivität: absolute $\Delta$ E405, gemessen 50 und 110 min nach Zugabe der Stimuli ohne Zellabtrennung, mit DYNATECH Microelisa Autoreader MR 580

| A | | | | | | C | | | | | |
|---|---|---|---|---|---|---|---|---|---|---|---|
| 0.25 | .170 | .147 | .058 | .165 | .030 | .015 | .085 | .033 | .025 | .033 | .022 |
| .032 | .180 | .163 | .070 | .155 | .040 | .018 | .095 | .043 | .028 | .039 | .027 |
| .018 | .177 | .154 | .066 | .168 | .037 | .013 | .098 | .037 | .029 | .027 | .029 |
| .039 | .187 | .170 | .084 | .175 | .054 | .022 | .087 | .033 | .031 | .026 | .015 |
| B .037 | .110 | .042 | .100 | .080 | .030 | D .010 | .220 | .170 | .015 | .130 | .240 |
| .027 | .105 | .040 | .095 | .090 | .025 | .017 | .205 | .185 | .025 | .135 | .235 |
| .045 | .103 | .026 | .092 | .095 | .038 | .019 | .235 | .165 | .022 | .138 | .224 |
| .047 | .122 | .045 | .087 | .094 | .024 | .025 | .217 | .175 | .027 | .125 | .238 |

Die vorstehende Tabelle zeigt die Meßwerte von 4 Probanden (Blöcke A-D), wobei die Zeilen 1-4 und 5-8 jeweils parallele Messungen zeigen. Die Spalten 1 und 7 zeigen die Kontrollwerte mit Pufferlösung, Spalten 2 und 8 die Induzierung mit anti-IgE-Antikörpern, Spalten 3 und 9 mit Allergenextrakt aus Gräserpollen (Allergopharma Ganzer KG, Best.-Nr. 006), Spalten 4 und 10 Allergenextrakt aus Haselnuß (Allergopharma Ganzer KG, Best.-Nr. 012), Spalten 5 und 11 Allergenextrakt aus Birkenpollen (Allergopharma Ganzer KG, Best.-Nr. 013) und Spalten 6 und 12 Allergenextrakt aus Milben (Allergopharma Ganzer KG, Best.-Nr. 725). Ach die Reaktion mit anti-IgE-Antikörpern ist bei Proband A, B und D ein Hinweis auf eine bestehende Allergie gegeben. Proband A zeigt eine deutlich allergische Reaktion mit Gräsern und Birkenpollen.

Proband B zeigt eine mittelstarke Reaktion mit beiden Baumpollen.

Proband D reagiert empfindlich auf Gräser, Birken und sehr stark mit Milbenallergen. Lediglich auf Haselnuß ist keine signifikante Reaktion zu sehen.

Der Allergieverdacht aus der anti-IgE-Reaktion wird durch die allergenspezifischen Reaktionen der Probanden A, B und D bestätigt.

Proband C reagiert deutlich schwächer mit anti-IgE und praktisch nicht mit allen Allergenen, er ist also nicht allergisch.

**Beispiel 5**

Hemmung der anti-IgE-induzierten Proteinasefreisetzung aus gepoolter Leukozytensuspension gesunder Probanden (n = 4) mit verschiedenen Standardsubstanzen

In einer Mikrotiterplatte (Nunclon 96U) mit 8 x 12 Gefäßen werden jeweils 0,1 ml gemäß Beispiel 1.1 präparierte Leukozyten vorgelegt (ca. $1,2 \times 10^7$ Zellen/ml) und jeweils 4 Gefäße mit 0,1 ml der zu testenden Substratlösung in den unten angegebenen Konzentrationen versetzt. Danach wird 0,1 ml Reagenzlösung aus $5 \times 10^{-4}$ M Chromozym TH, $3 \times 10^{-3}$ M $CaCl_2$, 0,15 M Trispuffer pH 7,6 in isotonischer wässriger NaCl-Lösung zugefügt, bei 37°C inkubiert und nach 45 min und 140 min ohne Zellabtrennung mit einem Dynatech Microeliser Autoreader MR 580 die Extinktion bei 405 nm gemessen.

Konzentration der Substrate bezogen auf Gesamtansatz:

Theophyllin: $\quad$ $10^{-3}$ M, $2 \times 10^{-4}$ M, $4 \times 10^{-5}$ M

Papaverin: $\quad$ $10^{-4}$ M, $2 \times 10^{-5}$ M, $4 \times 10^{-6}$ M

Cromoglicate: $\quad$ $5 \times 10^{-4}$ M, $\quad$ $10^{-4}$ M, $2 \times 10^{-5}$ M

Jodacetat: $\quad$ $5 \times 10^{-3}$ M, $\quad$ $10^{-3}$ M, $2 \times 10^{-5}$ M

2,4-Dinitrophenol: $5 \times 10^{-5}$ M, $\quad$ $10^{-5}$ M .

Picumast: $\quad$ $5 \times 10^{-5}$ M, $\quad$ $10^{-5}$ M, $2 \times 10^{-6}$ M

Es zeigt sich, daß als antiallergisch-wirksam bekannte Substanzen auch in der neuen Testanordnung eine deutliche, teilweise konzentrationsabhängige Hemmwirkung ergeben. Ebenso fördern auch Energie-stoffwechselblocker wie Dinitrophenol oder Jodacetat die Proteinasefreisetzung, nicht aber erwartungsgemäß der ß-Stimulator Isoprenalin oder Cromoglicate. Der Test ist somit für eine Reihenuntersuchung solcher Substanzen gut geeignet.

**Beispiel 6**

Nachweis von Allergenen mit Teststreifen

Filterpapier Schleicher und Schüll Nr. 2352 wird mit folgendem Gemisch getränkt, bei 60° C getrocknet und 10 mm breite Streifen geschnitten.

EP 0 185 335 B1

| 15 | m mol | Tris-Puffer pH 8.0 |
| 130 | m mol | Natriumchlorid |
| 0,2 | g | Gelatine MG ca. 10 000 |
| 50 | mg | Allergenmischung (Gräser, Bäume, Milben) |
| 2 | m mol | Calziumchlorid |
| 6 | m mol | Glucose |
| 1 | m mol | Indikator (gelöst in 10 ml Aceton) |
| 5 | m mol | Phosphorsäuretrimorpholid |
| 2 | g | Decanol |
| ad 1 | l | Wasser |

Als Indikatoren werden
3-(N-Succinyl)-L-alanyloxy-indol
3-(N-Toluol-sulfonyl-L-alanyloxy)-indol
3-(N-(Toluol-4-sulfonyl)-L-valyloxy)-indol
mit gleichem Ergebnis eingesetzt.

Diese Bänder werden mittels heißer Walzen zwischen einem 60 mm breiten Band schmelzwachsbeschichteter Polyesterfolie und einem 20 mm breiten Band Polyestervlies (15 g/m$^2$) so eingesiegelt, daß die Mitte des Testpapiers 6 mm von unterem Rand des Polyesterbandes und unter die Mitte des Vliesbandes zu liegen kommt, wobei die Heizwalzen an der Stelle des Testpapiers eine Aussparung besitzen. Das fertig versiegelte Band wird dann quer in 6mm breite Streifen geschnitten.

Taucht man diese Streifen in eine ca. 10$^7$ Leukozyten/ml haltige Suspension gemäß Beispiel 1 ein, dann erhält man mit Leukozyten von Allergikern nach 15-30 min blau bis dunkelblau verfärbte Papiere. Mit Leukozyten von gesunden Probanden oder mit reiner Pufferlösung verfärben sich die Papiere nicht oder nur blaßblau. Die Verfärbung kann gegebenenfalls auch remissionsphotometrisch vermessen werden.

## Ansprüche

1. Verfahren zum Nachweis des Vorliegens einer Allergie und zum spezifischen Nachweis des für die Allergie verantwortlichen Allergens in einem Schritt, bei dem Leukozyten der zu untersuchenden Probe mit einem Allergen oder mit einem anderen Stimulationsfaktor in wässrigem Medium inkubiert und die freigesetzte Protease mit einem Chromogen zur Reaktion gebracht und das entstehende Chromophor bestimmt wird, dadurch gekennzeichnet, daß das Allergen in einem Puffer mit einem pH von 6,3 bis 9 zusammen mit Calciumionen und Chromogen in isotonischer Lösung gemischt und mit den zu untersuchenden Leukozyten vermischt werden, so daß Calciumionen in einer Endkonzentration von 0,5 - 5 x 10$^{-3}$ mol/l, Chromogen in einer Endkonzentration von 10$^{-4}$ - 10$^{-3}$ mol/l und die Leukozyten in einer Endkonzentration von 10$^9$ - 10$^{10}$ pro Liter vorliegen und die Proteaseaktivität nach einer Inkubationszeit kinetisch an der Zunahme der Chromophorkonzentrationen gemessen wird.

2. Verfahren gemäß Anspruch 1, dadurch gekennzeichnet, daß der Puffer in einer Endkonzentration von 5 x 10$^{-3}$ bis 10$^{-1}$ mol/l im Testansatz vorliegt und einen pH-Wert von 7-8 besitzt.

3. Verfahren gemäß Anspruch 1 oder 2, dadurch gekennzeichnet, daß ein Trispuffer, Phosphatpuffer oder Glycinpuffer verwendet wird.

4. Verfahren gemäß Anspruch 1 bis 3, dadurch gekennzeichnet, daß die Lösung zusätzlich Magnesiumionen und/oder Gelatine oder andere Makromoleküle mit zellschützenden Eigenschaften enthält.

5. Verfahren gemäß einem der Ansprüche 1 bis 4, dadurch gekennzeichnet, daß anti-IgE Antikörper als unspezifischer Stimulator verwendet wird.

11

6. Verfahren gemaß einem der Ansprüche 1 bis 5 zum Nachweis von rheumatischer Arthritis, dadurch gekennzeichnet, daß Zellkernbestandteile eukaristischer Zellen, speziell RNS und DNS oder auch Immunkomplexe, als Stimulator verwendet werden.

7. Verfahren nach einem der Ansprüche 1-4, dadurch gekennzeichnet, daß ein Allergen, bzw. anderes Stimulans in einer Menge von 0,01 - 100 mg/l, insbesondere 1-10 mg/l, im Testansatz verwendet wird.

8. Verfahren nach einem der Ansprüche 1 bis 7, dadurch gekennzeichnet, daß die kinetische Messung nach einer Inkubationszeit von 15 bis 60 Minuten und einer Inkubationstemperatur zwischen 20 und 37°C durchgeführt wird.

9. Reagenz zum Nachweis von Allergien oder anderer entzündlicher Erkrankungen gemäß Ansprüchen 1-8, bestehend aus Puffer mit einem pH von 6,3 - 9, Allergen oder anderem Stimulans, Calciumsalz und Chromogen, sodaß das Allergen oder das andere Stimulans im Testansatz in einer Menge von 0,01 - 100 mg/l, insbesondere 1 - 10 mg/l, Calciumsalz in einer Endkonzentration von 0,5 - 5 x $10^{-3}$ mol/l und Chromogen in einer Endkonzentration von $10^{-4}$ - $10^{-3}$ mol/l vorliegen.

10. Kombination zum Nachweis von Allergien bzw. entsprechender Stimulationsfaktoren sowie der Bestimmung von antiallergischen Substanzen, dadurch gekennzeichnet, daß die Kombination aus einer Mikrotiterplatte besteht, die in Reihen angeordnet Reagenz gemäß Anspruch 9 mit anti-IgE Antikörpern bzw. verschiedenen Allergenen bzw. anderen Stimulanzien in verschiedenen Konzentrationen enthält, wobei in gegebenenfalls mehrfachen Positionen die gleichen Reagenzmischungen zur Mittelwertbildung vorgesehen sind.

11. Verfahren zur Bestimmung von Antiallergika und Antiinflammatorika , dadurch gekennzeichnet, daß man ein Reagenz gemäß Anspruch 9 in einem Verfahren gemäß einem der Ansprüche 1 - 8 mit dem Pharmakon versetzt und Leukozyten zusetzt, die gegen das Allergen oder weitere Stimulantien (anti-IgE-Antikörper, opsonisiertes Zymosan) sensibel sind und die gegenüber einer Kontrolle abgeschwächte Reaktion mißt.

12. Verfahren zur Bestimmung von Antiallergika und Antiinflammatorika, dadurch gekennzeichnet, daß man ein Reagenz gemäß Anspruch 9 in einem Verfahren gemäß einem der Ansprüche 1 bis 8 mit Leukozyten versetzt, welche vorher 5-60, insbesondere 30 min, bei 20-37°C mit den zu prüfenden Pharmaka vorinkubiert werden

13. Verfahren zur Bestimmung von Antiallergika und Antiinflammatorika gemäß Anspruch 11 oder 12, dadurch gekennzeichnet, daß man die Leukozytensuspension verteilt in einzelne Reaktionsgefäße vorlegt, mit den zu prüfenden Pharmaka versetzt und gemäß Anspruch 12 vorinkubiert sowie nachfolgend die Farbreaktion auslöst durch Zugabe eines Reagenz gemäß Anspruch 9.

**Claims**

1. Process for the detection of the presence of an allergy and for the specific detection of the allergen responsible for the allergy in one step, in which leukocytes of a sample to be investigated are incubated with an allergen or with another stimulation factor in aqueous medium and the liberated protease is brought to reaction with a chromogen and the resulting chromophor is determined, characterised in that the allergen is mixed in a buffer with a pH of 6.3 to 9 together with calcium ions and chromogen in isotonic solution and mixed with the leukocytes to be investigated so that calcium ions are present in an end concentration of 0.5 - 5 x $10^{-3}$ mol/l., chromogen in an end concentration of $10^{-4}$ - $10^{-3}$ mol/l. and the leukocytes in an end concentration of $10^9$ - $10^{10}$ per litre and the protease activity is measured kinetically after an incubation period by the increase of the chromophor concentrations.

2. Process according to claim 1, characterised in that the buffer is present in the test batch in a concentration of from 5 x $10^{-3}$ to $10^{-1}$ mol/l. and possesses a pH value of 7 - 8.

3. Process according to claim 1 or 2, characterised in that a tris buffer, phosphate buffer or glycine buffer is used.

4. Process according to claim 1 to 3, characterised in that the solution additionally contains magnesium ions and/or gelatine and other macromolecules with cell-protecting properties.

5. Process according to one of claims 1 to 4, characterised in that anti-IgE antibody is used as non-specific stimulator.

6. Process according to one of claims 1 to 5 for the detection of rheumatic arthritis, characterised in that cell nucleus parts of eukaryotic cells, especially RNA or DNA, or also immune complexes are used as stimulator.

7. Process according to one of claims 1 - 4, characterised in that an allergen or other stimulant is used in the test batch in an amount of 0.01 - 100 mg./l., especially 1 - 10 mg./l.

8. Process according to one of claims 1 - 7, characterised in that the kinetic measurement is carried out after an incubation time of from 15 to 60 minutes and at an incubation temperature between 20 and 37°C.

9. Reagent for the detection of allergies or of other inflammatory diseases according to claims 1 - 8, consisting of a buffer with a pH of 6.3 - 9, allergen or other stimulant, calcium salt and chromogen so that the allergen or other stimulant is present in the test batch in an amount of 0.01 to 100 mg./l., especially of 1 - 10 mg./l., calcium salt in an end concentration of $0.5 - 5 \times 10^{-3}$ mol/l. and chromogen in an end concentration of $10^{-4}$ to $10^{-3}$ mol/l.

10. Combination for the detection of allergies or corresponding stimulation factors, as well as the determination of anti-allergic substances, characterised in that the combination consists of a microtitre plate which contains, arranged in rows, reagent according to claim 9 with anti-IgE antibodies or various allergens or other stimulants in various concentrations, whereby the same reagent mixtures are possibly provided in several positions for obtaining average values.

11. Process for the determination of anti-allergic and anti-inflammatory substances, characterised in that one mixes a reagent according to claim 9 in a process according to one of claims 1 - 8 with the pharmaceutical and adds leukocytes thereto which are sensitive towards the allergen or further stimulants (anti-IgE antibodies, opsonised zymosan) and measures the reaction weakened in comparison with a control.

12. Process for the determination of anti-allergic and anti-inflammatory substances, characterised in that one mixes a reagent according to claim 9 in a process according to one of claims 1 to 8, with leukocytes which have been previously pre-incubated for 5 - 60, especially 30 min. at 20 - 37°C. with the pharmaceutical to be tested.

13. Process for the determination of anti-allergic and anti-inflammatory substances according to claim 11 or 12, characterised in that one takes the leukocyte suspension distributed in individual reaction vessels, mixes with the pharmaceutical to be tested and pre-incubates according to claim 12, as well as subsequently initiates the colour reaction by addition of a reagent according to claim 9.

**Revendications**

1. Procédé pour la détection de la présence d'une allergie et pour la détection spécifique des allergènes responsables de l'allergie, en une étape, dans lequel les leucocytes de l'échantillon à examiner sont incubés avec un allergène ou avec un autre facteur stimulant, en milieu aqueux, et la protéase libérée est mise à réagir avec un chromogène et le chromophore formé est déterminé, caractérisé en ce que l'allergène est mélangé dans un tampon à pH 6,3 à 9, conjointement avec des ions calcium et un chromogène en solution isotonique et avec les leucocytes à examiner, de sorte que les ions calcium se trouvent à une concentration finale de 0,5 à $5 \times 10^{-3}$ mole/l, le chromogène, à une concentration finale de $10^{-4}$ à $10^{-3}$ mole/l et les leucocytes, à une concentration finale de $10^{9}$ à $10^{10}$ par litre et que l'activité de la protéase, après un temps d'incubation, est mesurée cinétiquement par l'accroissement de la concentration en chromophore.

2. Procédé selon la revendication 1, caractérisé en ce que le tampon est présent dans le produit à tester en une concentration finale de $5 \times 10^{-3}$ à $10^{-1}$ mole/l et présente un pH de 7 à 8.

3. Procédé selon la revendication 1 ou 2, caractérisé en ce que l'on utilise un tampon tris, un tampon phosphate ou un tampon glycine.

4. Procédé selon les revendications 1 à 3, caractérisé en ce que la solution contient en outre des ions magnésium et/ou de la gélatine ou d'autres macromolécules ayant des propriétés protectrices de la cellule.

5. Procédé selon l'une quelconque des revendications 1 à 4, caractérisé en ce que des anticorps anti-IGE sont utilisés comme stimulants non spécifiques.

6. Procédé selon l'une quelconque des revendications 1 à 5, pour la détection de l'arthrite rhumatoïde, caractérisé en ce que comme stimulant on utilise des constituants du noyau cellulaire de cellules eucariotiques en particulier l'ARN ou l'ADN, ou encore des immunocomplexes.

7. Procédé selon l'une quelconque des revendications 1 à 4, caractérisé en ce que dans la matière à tester, on utilise un allergène, ou un autre stimulant, en une quantité de 0,01 à 100 mg/l, en particulier 1 à 10 mg/l.

8. Procédé selon l'une quelconque des revendications 1 à 7, caractérisé en ce que la mesure cinétique est effectuée après un temps d'incubation de 15 à 60 minutes et à une température d'incubation comprise entre 20 et 37° C.

9. Réactif pour la détection d'allergies ou d'autres affections inflammatoires, selon les revendications 1 à 8, constitué d'un tampon ayant un pH de 6,3 à 9, d'un allergène ou d'un autre stimulant, de sels de calcium et d'un chromogène, tel que l'allergène ou l'autre stimulant sont présents dans le milieu à tester en une quantité de 0,01 à 100 mg/l, en particulier 1 à 10 mg/l, le sel de calcium est présent en une concentration finale de 0,5 à $5 \times 10^{-3}$ mole/l et le chromogène est présent en une concentration finale de $10^{-4}$ à $10^{-3}$ mole/l.

10. Combinaison pour la détection d'allergies ou des facteurs de stimulation correspondants, ainsi que pour la détermination de substances anti-allergiques, caractérisée en ce que la combinaison est constituée d'une plaque de microtitrage, qui contient des réactifs rangés dans l'ordre selon la revendication 9, avec des anticorps anti-IGE ou divers allergènes ou d'autres stimulants, en diverses concentrations, les mêmes mélanges de réactifs étant prévus éventuellement en plusieurs positions pour déterminer une valeur moyenne.

11. Procédé pour la détermination d'agents anti-allergiques et d'agents anti-inflammatoires, caractérisé en ce qu'à un réactif selon la revendication 9 on ajoute, dans un procédé selon l'une quelconque des revendications 1 à 8, le médicament et les leucocytes qui sont sensibles à l'allergène ou à d'autres stimulants (anticorps anti-IGE, zymosan opsonisé) et l'on mesure la réaction affaiblie par rapport à un témoin.

12. Procédé pour la détermination d'agents anti-allergiques et d'agents anti-inflammatoires, caractérisé en ce qu'à un réactif selon la revendication 9 on ajoute, dans un procédé selon l'une quelconque des revendications 1 à 8, des leucocytes qui ont été incubés préalablement pendant 5 à 60 mn, en particulier 30 mn, à une température de 20 à 37° C, avec le médicament à tester.

13. Procédé pour la détermination d'agents anti-allergiques et d'agents anti-inflammatoires, selon les revendications 11 et 12, caractérisé en ce que l'on répartit la suspension de leucocytes dans un récipient réactionnel unique, l'on y ajoute le médicament à tester et le soumet à une pré-incubation selon la revendication 12 ainsi qu'à une réaction de coloration subséquente, par addition d'un réactif selon la revendication 9.